# EUROPÄISCHE PATENTSCHRIFT

(11) **EP 1 905 358 B1**
(45) Veröffentlichungstag und Bekanntmachung des Hinweises auf die Patenterteilung: **14.12.2016**
(21) Anmeldenummer: 07117459.3
(22) Anmeldetag: 28.09.2007
(51) Int. Cl.: A61B 6/14, A61B 6/04

(54) **Positioniervorrichtung eines Patienten an einem Panorama-Röntgengerät**
Patient positioning device of a panoramic dental x-ray apparatus
Ensemble de porte ou de fenêtre avec inserts vitrés

(30) Priorität: 28.09.2006 DE 102006046271
(43) Veröffentlichungstag der Anmeldung: 02.04.2008
(73) Patentinhaber: Sirona Dental Systems GmbH, 64625 Bensheim (DE)
(72) Erfinder: Pruß, Malte, 64665, Alsbach-Hähnlein (DE); Döbert, Michael, 64653, Lorsch (DE)
(74) Vertreter: Sommer, Peter

(56) Entgegenhaltungen:
- DE-A1- 19 910 107
- DE-A1-102004 041 440
- JP-A- 2 093 452
- JP-U- 54 028 374
- US-A- 3 521 057
- US-A- 3 539 805
- US-A- 3 936 641
- US-A- 4 147 662
- US-A- 4 974 243
- US-A- 5 921 927

## Beschreibung

### Technisches Gebiet

Die Erfindung betrifft eine Positioniervorrichtung zur korrekten Positionierung eines Patienten bei einer Aufnahme mit einem medizinischen Panorama-Röntgenaufnahmegerät.

### Stand der Technik

Bei einem Panorama-Röntgengerät muss der Bediener den Patienten zur Erstellung einer Aufnahme und gegebenenfalls auch während der Aufnahme positionieren. Dies geschieht mit einer Positioniervorrichtung, die ein Aufbissteil oder ein Anlageteil umfasst. Für unterschiedliche Aufnahmearten muss dabei das Positionierteil gewechselt werden, um eine Positionierung in unterschiedlichen Höhen zu ermöglichen. Durch das Wechseln kann trotz aller Vorsorgemaßnahmen eine Verwendung des nicht für die Aufnahme geeigneten Aufbissteils nicht ausgeschlossen werden. Zusätzlich sind für frontzahnlose Patienten weitere Anlagestücke notwendig, etwa ein subnasales Anlagestücke, auf das der Patient nicht aufbeisst, sondern an das er angelehnt wird.

Aus der DE 36 09 260 A1 ist ein Panorama-Röntgengerät zur Erstellung einer Panorama-Röntgenaufnahme bekannt, welches drei auswechselbare Aufbissteile aufweist. Ein erstes Aufbissteil ist für sogenannte Standardaufnahmen vorgesehen, ein zweites Aufbissteil ist für sogenannte Sinus- und Kiefergelenk-Aufnahmen geeignet. Ein drittes Aufbissteil ist ebenfalls für Kiefergelenkaufnahmen geeignet, jedoch aus einer anderen Durchstrahlungsrichtung. Die Aufbissteile weisen einen Sockel auf, der gleich ausgebildet ist, sodass alternativ das eine oder andere Aufbissteil in einen Halte- und Führungsschaft eines Trägers eingesetzt werden kann. Die Aufbissteile enthalten zur Unterscheidung voneinander unterschiedliche Kennungen, die insbesondere als jeweils eine farbige Markierung ausgeführt sind.

Aus der DE 35 30 234 A1 ist ein Aufbissteil vorgesehen, das an einen um das entsprechende Höhen- und Tiefenmaß verstellbaren Träger angeordnet ist. Der Träger ist als exzentrisch gelagerte Walze ausgebildet und weist zwei Aufnahmeöffnungen für den Träger auf, die für eine StandardAufnahme bzw. für eine Sinus- und Kiefergelenkaufnahme vorgesehen sind.

Aus der DE 102 50 005 A1 ist eine Aufbissvorrichtung bekannt, die eine an einem Halteteil angebrachte schwenkbare Platte aufweist. Eine Höhenanpassung des Aufbisses ist damit aber nicht gegeben, da lediglich der Neigungswinkel der Kauebene erfasst bzw. das Röntgengerät auf einen vorgegebenen Wert eingestellt werden soll.

Es kommt aber vor, dass bei den vorgegebenen Positionen bei der Erstellung von transversalen Schichtaufnahmen (TSA) von Backenzähnen keine vollständige Abbildung des Ramus, d.h. der unteren Kante des Unterkiefers erfolgt.

Auch Aufnahmen im Mittelgesicht, d.h. oberhalb des Oberkiefers sind zum Teil unvollständig, besonders die Kiefergelenke.

Aus der WO 02/086619 A1 ist ein Schichtbildaufnahmegerät für dentale Tomosynthese bekannt, bei dem ein Bissstück an einem Gelenkarm befestigt ist, wobei der Gelenkarm mit einem Knebel fixiert wird, wenn sich der Patient in der richtigen Position befindet. Nachteilig hierbei ist, dass keine Standardpositionen eingestellt werden können.

Der Erfindung, wie sie in den Ansprüchen gekennzeichnet ist, liegt die Aufgabe zugrunde, eine Vorrichtung zum korrekten Positionieren eines Patienten bei einer Aufnahme mit einem medizinischen Panorama-Röntgenaufnahmegerät anzugeben, die eine einfache und robuste Positionierung eines Patienten erlaubt.

### Darstellung der Erfindung

Diese Aufgabe wird erfindungsgemäß durch die Positioniervorrichtung nach Anspruch 1 gelöst. Vorteilhafte Ausgestaltungen der Erfindung sind Gegenstand der Unteransprüche.

Der Vorteil besteht darin, dass der Anwender auch für verschiedene Aufnahmen das Positionierteil nicht wechseln muss, sondern lediglich eine Höhenverstellung durchführt. Anstelle der bislang verwendeten drei Aufbissteile, nämlich normaler Aufbiss und zwei Aufbisselemente für die Mittelgesichtsaufnahme bzw. drei Anlagenteile kann ein einziger Aufbiss bzw. ein einziges Anlagenteil verwendet werden. Die Gefahr, dass ein Austausch des Positionierteils zur Anpassung der Größe unterbleibt, ist damit verringert.

Weitere vorteilhafte Ausgestaltungen, Merkmale und Details der Erfindung ergeben sich aus den abhängigen Ansprüchen, der Beschreibung der Ausführungsbeispiele und den Zeichnungen.

Vorteilhafterweise weist das Halterteil einen Sockel zur Befestigung an einem Träger des Röntgengeräts auf und ist die Längsführung zu dem Sockel axial beabstandet, vorteilhafterweise in Richtung zum Patienten hin. Dadurch ist es möglich, das Positionierteil bei der Längsverschiebung an dem Träger vorbeizuführen.

Vorteilhafterweise können in der Längsführung für vorgegebene Standardhöhen Rastungen entsprechend eines Normalkiefers für das Positionierteil vorgesehen sein. Damit wird eine schnelle und sichere Festlegung der richtigen Höhe ermöglicht.

Vorteilhafterweise können in der Längsführung zur Fixierung des Positionierteils in außerhalb der Rastungen liegenden Positionen Feststellmittel vorgesehen sein. Dadurch sind individuelle Anpassungen an anatomische Besonderheiten des Patienten möglich.

Vorteilhafterweise können die Standardhöhen des Positionierteils am Positionierteil mittels Markierungen markiert sein, sodass Fehleinstellungen weitgehend vermeidbar sind.

Insbesondere geeignet sind eindeutig farblich gegeneinander abgesetzte Markierungen.

### Kurzbeschreibung der Zeichnung

Die Erfindung wird anhand eines Ausführungsbeispiels erläutert. Dabei zeigt:
- Fig. 1: eine schematische Darstellung einer PanoramaRöntgenaufnahmeeinrichtung mit einer Positioniervorrichtung mit einem Positionierteil in Form eines Aufbissteils oder eines Anlageteils,
- Fig. 2: eine Positioniervorrichtung mit einem Aufbissteil,
- Fig. 3: eine Positioniervorrichtung mit einem Anlageteil.

### Ausführungsbeispiel

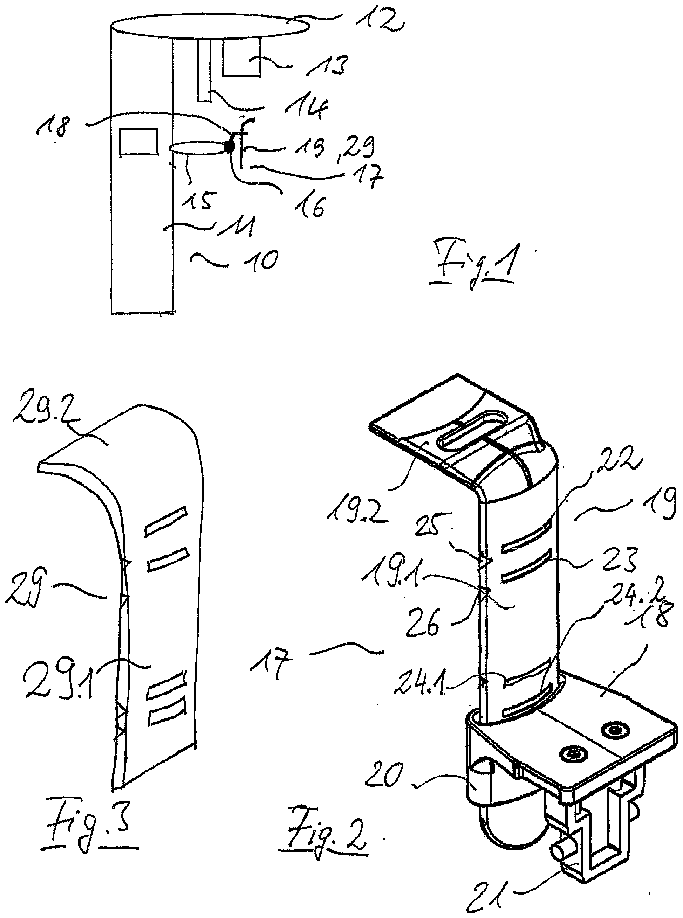

Fig. 1 zeigt in schematischer Darstellung eine Panorama-Röntgenaufnahmeeinrichtung 10, bei der eine an einer Säule 11 angeordnete schwenkbare Einheit 12 eine Strahlenquelle 13 und eine gegenüberliegende Detektorkamera 14 trägt. An der Säule 11 ist darüber hinaus ein Träger 15 mit einem Halter 16 für eine Positioniervorrichtung 17 mit einem Halterteil 18 für ein gegenüber dem Halterteil 18 höhenverstellbares Positionierteilteil 19.

Die Anpassung des Geräts 10 an die Körpergröße des Patienten erfolgt durch eine Höhenverstellung der Dreheinheit 12 gegebenenfalls zusammen mit der Höhenverstellung des Trägers 15. Die Anpassung an die Aufnahmeart erfolgt durch eine Einstellung der Positioniervorrichtung 17.

Fig. 2 zeigt eine perspektivische Ansicht der Positioniervorrichtung 17 nach einem Ausführungsbeispiel der Erfindung mit einem Aufbissteil 19. Die Positioniervorrichtung 17 weist ein Halteteil 18 auf, an dem das Aufbissteil 19 mittels einer Längsführung 20 längsverschieblich geführt ist. Das Aufbissteil 19 weist dazu einen geführten Einstellabschnitt 19.1 und eine demgegenüber abgewinkelte Aufbissplatte 19.2 auf.

Die Längsführung 20 ist zu einem Sockel 21 axial zum Patienten hin beabstandet und umgreift den Einstellabschnitt 19.1 des Aufbissteils 19 zumindest teilweise. Der Einstellabschnitt 19.1 enthält Rastungen für eine Arretierung in der Längsführung 20 in vorgegebenen Standardhöhen, die auf einen Normalkiefer ausgelegt sind. Um eine eindeutige Rastposition zu ermöglichen kann eine federnde Zunge an der Längsführung 20 in eine Nut 25, 26 an dem Einstellabschnitt 19.1 eingreifen. Zwischenpositionen werden in der Regel nicht benötigt, können aber durch einen Klemmmechanismus sichergestellt werden, in dem von unten bzw. oben am Aufbissteil 19 zwei Teile gegen das Halteteil 18 geschoben werden.

Am Einstellabschnitt 19.1 sind drei Markierungen 22-24.1 sowie eine Markierung 24.2 in verschiedenen Höhen vorgesehen, denen Rastpositionen des Einstellabschnitts 19.1 in der Längsführung entsprechen können. Die Markierungen 22-24.2 können bisher standardmäßig benutzten verschiedenen Aufbisselementen für die Standardaufnahme, für die Sinus- und Kiefergelenkaufnahme und für eine weitere Kiefergelenkaufnahme aus einer anderen Durchstrahlrichtung entsprechen.

Dabei dient die oberste Markierung 22 zur Positionierung des Patienten für die Erstellung einer Transversalschichtaufnahme des Mittelgesichts, auch als Mittelgesichtsaufnahme oder Sinusaufnahme bezeichnet, und des Oberkiefers, auch als Oberkieferaufnahme bezeichnet.

Die zweitoberste Markierung 23 dient zur Positionierung des Patienten für die Erstellung einer Kiefergelenksaufnahme. Darüber hinaus kann in dieser Position auch eine Mittelgesichtsaufnahme oder Sinusaufnahme und eine Oberkieferaufnahme erstellt werden. Diese Aufnahmen sind ebenfalls Transversalschichtaufnahmen.

Die zweitunterste Markierung 24.1 dient zur Positionierung des Patienten für die Erstellung einer Standard-Panoramaschichtaufnahme sowie anderen Standardaufnahmen. Darüber hinaus kann in dieser Position auch eine Transversalschichtaufnahme des Unterkiefers erstellt werden, auch als Unterkieferaufnahme bezeichnet.

Die unterste Markierung 24.2 dient zur Positionierung des Patienten für die Erstellung einer Transversalschichtaufnahme des Unterkiefers in einer noch tieferen Position.

Um die Gefahr von Fehlpositionierungen zu verhindern, können die Markierungen 22-24.2 farblich voneinander abgesetzt sein.

In Fig. 3 ist ein subnasales Anlagestück 29 für frontzahnlose Patienten dargestellt, welches einen Einstellabschnitt 29.1 aufweist, der etwas länger als beim Aufbissteil 19 aus Fig. 2 ist, sodass ein Anlageabschnitt 29.2 höher liegt als die Aufbissplatte 19.2. Das Anlagestück 29 ist ebenfalls farbcodiert und entspricht in seinem Verbindungsbereich dem Aufbissteil 19 aus Fig. 2, sodass es anstelle des Aufbissteils 19 in den Halter 20 einsteckbar ist.

## Patentansprüche

1. Positioniervorrichtung eines Panorama-Röntgengeräts (10) zur Positionierung eines Patienten in dem Panorama-Röntgengerät (10) mit einem zum Röntgengerät (10) richtungsfest anordenbarem Halterteil (18) und einem mit dem Halteteil (18) verbundenen verstellbaren Positionierteil (19, 29), wobei das Halterteil (18) eine Längsführung (20) für das Positionierteil (19, 29) zur Höhenverstellung des Positionierteils (19, 29) gegenüber dem Halterteil (18) aufweist, **dadurch gekennzeichnet, dass** das Positionierteil (19, 29) Rastungen für eine Arretierung in der Längsführung (20) in vorgegebenen Standardhöhen entsprechend eines Normalkiefers aufweist, wobei eine oberste Standardhöhe zur Positionierung des Patienten zur Erstellung einer Mittelgesichtsaufnahme und einer Oberkieferaufnahme dient, eine zweitoberste Standardhöhe zur Positionierung des Patienten zur Erstellung einer Kiefergelenksaufnahme dient, eine zweitunterste Standardhöhe zur Positionierung zur Erstellung einer Standard-Panoramaschichtaufname und einer Unterkieferaufnahme dient und eine unterste Standardhöhe zur Positionierung zur Erstellung einer Unterkieferaufnahme dient.

2. Positioniervorrichtung eines Panorama-Röntgengeräts (10) zur Positionierung eines Patienten in dem Panorama-Röntgengerät (10) mit einem zum Röntgengerät (10) richtungsfest anordenbarem Halterteil (18) und einem mit dem Halteteil (18) verbundenen verstellbaren Positionierteil (19, 29), wobei das Halterteil (18) eine Längsführung (20) für das Positionierteil (19, 29) zur Höhenverstellung des Positionierteils (19, 29) gegenüber dem Halterteil (18) aufweist, **dadurch gekennzeichnet, dass** das Positionierteil (19, 29) Rastungen für eine Arretierung in der Längsführung (20) in vorgegebenen Standardhöhen entsprechend eines Normalkiefers aufweist und dass in der Längsführung (15) zur Fixierung des Positzonierteils (19, 29) in außerhalb der Rastungen liegenden Positionen Feststellmittel vorgesehen sind.

3. Positioniervorrichtung nach Anspruch 1 oder 2, **dadurch gekennzeichnet, dass** das Positionierteil ein Aufbissteil (19) oder ein Anlageteil (29) ist.

4. Positioniervorrichtung nach einem der Ansprüche 1 bis 3, **dadurch gekennzeichnet, dass** das Halterteil (18) einen Sockel (21) zur Befestigung an einem Träger (15) des Röntgengeräts (10) aufweist und dass die Längsführung (20) zu dem Sockel (21) axial beabstandet ist.

5. Positioniervorrichtung nach einem der Ansprüche 1 und 3 bis 4, **dadurch gekennzeichnet, dass** in der Längsführung (15) zur Fixierung des Positionierteils (19, 29) in außerhalb der Rastungen liegenden Positionen Feststellmittel vorgesehen sind.

6. Positioniervorrichtung nach einem der Ansprüche 1 bis 5, **dadurch gekennzeichnet, dass** unterschiedliche Standardhöhen am Positionierteil (19, 29) mittels Markierungen (22-24.2) markiert sind.

7. Positioniervorrichtung nach Anspruch 6, **dadurch gekennzeichnet, dass** die Markierungen (22-24.2) eindeutig farblich gegeneinander abgesetzt sind.

## Claims

1. A positioning device of a panoramic X-ray device (10) for positioning a patient in the panoramic X-ray device (10), with a retaining part (18) that can be arranged in a fixed direction with respect to the X-ray device (10), and with an adjustable positioning part (19, 29) that is connected to the retaining part (18), wherein the retaining part (18) comprises a longitudinal guide (20) for the positioning part (19, 29) for adjusting the height of the positioning part (19, 29) with respect to the retaining part (18), **characterized in that** the positioning part (19, 29) comprises catches for locking in the longitudinal guide (20) at predefined standard heights according to an average jaw, wherein an uppermost standard height is used to position the patient for taking a central face image and an upper jaw image, a second uppermost standard height is used to position the patient for taking a temporomandibular joint image, a second bottommost standard height is used for positioning for taking a standard panoramic tomographic image and a lower jaw image, and a bottommost standard height is used for positioning for taking a lower jaw image.

2. A positioning device of a panoramic X-ray device (10) for positioning a patient in the panoramic X-ray device (10), with a retaining part (18) that can be arranged in a fixed direction with respect to the X-ray device (10), and with an adjustable positioning part (19, 29) that is connected to the retaining part (18), wherein the retaining part (18) comprises a longitudinal guide (20) for the positioning part (19, 29) for adjusting the height of the positioning part (19, 29) with respect to the retaining part (18), **characterized in that** the positioning part (19, 29) comprises catches for locking in the longitudinal guide (20) at predefined standard heights according to an average jaw, and that locking means are provided in the longitudinal guide (15) for fixing the positioning part (19, 29) in positions outside of the catches.

3. The positioning device according to claim 1 or 2, **characterized in that** the positioning part is a bite part (19) or a contact part (29).

4. The positioning device according to one of claims 1 to 3, **characterized in that** the retaining part (18) comprises a base (21) for mounting on a support (15) of the X-ray device (10), and that the longitudinal guide (20) is axially spaced apart from the base (21).

5. The positioning device according to one of claims 1 and 3 to 4, **characterized in that** locking means are provided in the longitudinal guide (15) for fixing the positioning part (19, 29) in positions outside of the catches.

6. The positioning device according to one of claims 1 to 5, **characterized in that** different standard heights are marked on the positioning part (19, 29) by means of markings (22-24.2).

7. The positioning device according to claim 6, **characterized in that** the markings (22-24.2) have clearly contrasting colors.

## Revendications

1. Dispositif de positionnement d'un appareil de radiographie panoramique (10) pour le positionnement d'un patient dans l'appareil de radiographie panoramique (10) avec une partie support (18) pouvant être placée dans une direction fixe par rapport à l'appareil de radiographie (10) et une partie de positionnement (19, 29) pouvant être réglée reliée à la partie support (18), dans lequel la partie support (18) présente un guide longitudinal (20) pour la partie de positionnement (19, 29) pour le réglage en hauteur de la partie de positionnement (19, 29) par rapport à la partie support (18), **caractérisé en ce que** la partie de positionnement (19, 29) présente des cliquets pour un blocage dans le guide longitudinal (20) à des hauteurs classiques prédéfinies, correspondant à une mâchoire normale, dans lequel une hauteur classique la plus haute sert au positionnement du patient pour la réalisation d'une radiographie de la partie centrale du visage et d'une radiographie de la mâchoire supérieure, une deuxième hauteur classique la plus haute sert au positionnement du patient pour la réalisation d'une radiographie de l'articulation de la mâchoire, une deuxième hauteur classique la plus basse sert au positionnement pour la réalisation d'un orthopantomogramme classique et d'une radiographie de la mâchoire inférieure et une hauteur classique la plus basse sert au positionnement pour la réalisation d'une radiographie de la mâchoire inférieure.

2. Dispositif de positionnement d'un appareil de radiographie panoramique (10) pour le positionnement d'un patient dans l'appareil de radiographie panoramique (10) avec une partie support (18) pouvant être placée dans une direction fixe par rapport à l'appareil de radiographie (10) et une partie de positionnement (19, 29) pouvant être réglée reliée à la partie support (18), dans lequel la partie support (18) présente un guide longitudinal (20) pour la partie de positionnement (19, 29) pour le réglage en hauteur de la partie de positionnement (19, 29) par rapport à la partie support (18), **caractérisé en ce que** la partie de positionnement (19, 29) présente des cliquets pour un blocage dans le guide longitudinal (20) à des hauteurs classiques prédéfinies correspondant à une mâchoire normale et **en ce que**, dans le guide longitudinal (15), des moyens de blocage sont prévus pour la fixation de la partie de positionnement (19, 29) dans des positions se trouvant en dehors des cliquets.

3. Dispositif de positionnement selon la revendication 1 ou 2, **caractérisé en ce que** la partie de positionnement est une partie à mordre (19) ou une partie d'appui (29).

4. Dispositif de positionnement selon une des revendications 1 à 3, **caractérisé en ce que** la partie support (18) présente un socle (21) pour la fixation sur un support (15) de l'appareil de radiographie (10) et **en ce que** le guide longitudinal (20) est écarté axialement du socle (21).

5. Dispositif de positionnement selon une des revendications 1 et 3 à 4, **caractérisé en ce que** des moyens de blocage sont prévus dans le guide longitudinal (15) pour la fixation de la partie de positionnement (19, 29) dans des positions se trouvant en dehors des cliquets.

6. Dispositif de positionnement selon une des revendications 1 à 5, **caractérisé en ce que** des hauteurs classiques différentes sont marquées sur la partie de positionnement (19, 29) à l'aide de repères (22-24.2).

7. Dispositif de positionnement selon la revendication 6, **caractérisé en ce que** les repères (22-24.2) sont distingués les uns des autres clairement par coloration.
